# EUROPÄISCHE PATENTSCHRIFT

(11) **EP 0 789 011 B1**
(45) Veröffentlichungstag und Bekanntmachung des Hinweises auf die Patenterteilung: **26.04.2000**
(21) Anmeldenummer: 96120043.3
(22) Anmeldetag: 13.12.1996
(51) Int. Cl.: C07C 17/08, C07C 23/02

(54) **Verfahren zur Herstellung von Alkylhalogeniden**
Process for the preparation of alkyl halides
Procédé pour la préparation d'halogénures d'alkyle

(30) Priorität: 08.02.1996 DE 19604566
(43) Veröffentlichungstag der Anmeldung: 13.08.1997
(73) Patentinhaber: Degussa-Hüls Aktiengesellschaft, 60287 Frankfurt am Main (DE)
(72) Erfinder: Metz, Josef, Dr., 45770 Marl (DE); Osterholt, Clemens, 46286 Dorsten (DE)

(56) Entgegenhaltungen:
- RESEARCH DISCLOSURE, Nr. 355, 1.November 1993, Seite 754 XP000421384 "HYDROHALOGENATION OF CYCLOPENTENE"

## Beschreibung

Die Erfindung betrifft ein neues Verfahren zur Herstellung von Alkylhalogeniden aus cycloaliphatischen Olefinen mit 5 bis 16 C-Atomen und konzentrierter Halogenwasserstoffsäure.

Alkylhalogenide dienen als Lösemittel. Sie werden darüber hinaus für Friedel-Crafts-Alkylierungen, zur Herstellung metallorganischer Verbindungen und zur Synthese von Pflanzenschutzmitteln und pharmazeutischen Produkten verwendet.

Bei der Herstellung der Alkylhalogenide geht man im allgemeinen von Alkoholen oder von Olefinen aus.

Nach Tetrahedron 23, 2051 (1967), war die Herstellung von Cyclooctylchlorid aus Cycloocten und Salzsäure in Gegenwart von Zinkchlorid in Benzol als Lösemittel bekannt.

Gemäß JP 89/166 393 werden für die Herstellung cycloaliphatischer Alkylchloride AlCl₃, FeCl₃, NiCl₂ oder CuCl als Katalysator und Methylenchlorid, Chloroform oder Tetrachlorkohlenstoff als Lösemittel verwendet.

Die genannten Verfahren sind umständlich bzw. aufwendig, weil Lösemittel abgetrennt und Katalysatoren aufgearbeitet werden müssen.

Aufgabe der vorliegenden Erfindung war es daher, ein vereinfachtes Verfahren zur Herstellung von Alkylhalogeniden aus cycloaliphatischen Olefinen bereitzustellen.

Die Aufgabe wird erfindungsgemäß dadurch gelöst, daß man die Reaktion ohne Katalysatorzusatz und ohne Lösemittel bei einer Temperatur unterhalb des Siedepunkts des Olefins durchführt.

Geeignete cycloaliphatische Olefine sind beispielsweise Cyclopenten, Cyclohexen, Cyclohepten, Cycloocten, 1-Methylcyclopenten-1, 1,2-Dimethylcyclohexen-1, 1,4-Dimethylcycloocten-1 oder 1,4-Cyclooctadien. Vorzugsweise werden Cycloolefine mit 5 bis 10 C-Atomen eingesetzt.

Als Halogenwasserstoffsäuren kommen Chlor-, Brom- und Jodwasserstoffsäure in Betracht. Konzentrierte Halogenwasserstoffsäuren im Sinne dieser Erfindung sind mindestens 20%ige Säuren. Vorzugsweise setzt man 20- bis 45%ige Salzsäure ein.

Bevorzugt werden demnach cyclische Alkylchloride hergestellt.

Die Reaktionstemperatur richtet sich nach dem Siedepunkt des eingesetzten Olefins. Dabei liegt die Reaktionstemperatur vorzugsweise im Bereich von 0 bis 110 °C.

Neben einer Batch-Fahrweise ist auch eine kontinuierliche Verfahrensführung möglich.

Ein allgemeiner Vorteil des Verfahrens ist, daß die Reaktion bei Normaldruck durchgeführt werden kann. Überraschenderweise werden nach diesem Verfahren Alkylhalogenide bei hohen Olefinumsätzen, in hoher Reinheit und Selektivität und mit sehr guter Farbe erhalten. Man benötigt keine alkalische Wäsche. Es sind keine oder nur sehr einfache destillative Reinigungsoperationen zur weiteren Farbverbesserung erforderlich.

Die folgenden Beispiele sollen die Erfindung erläutern.

### Beispiel 1

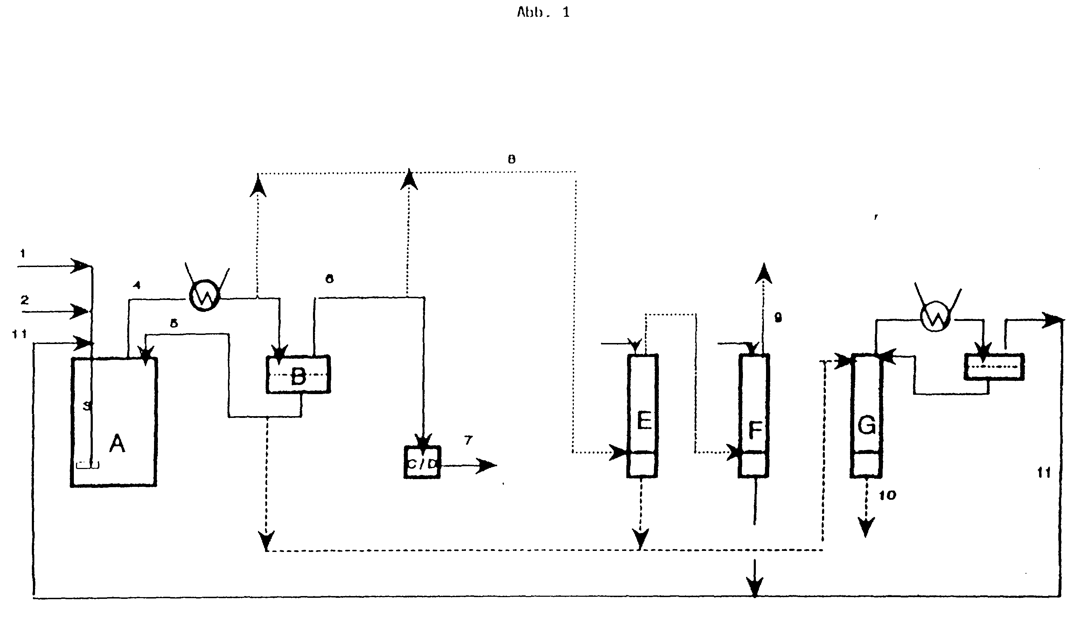

### Herstellung von Cyclopentylchlorid (Abb. 1)

In einem mit N₂ abgedeckten Glasreaktor (A) werden 100 g konzentrierte Salzsäure (ca. 36%ig) vorgelegt, auf 10 °C eingestellt und 340 g Cyclopenten technisch (Gehalt GC: 96,7 %, 3,2 % Cyclopentan) zugefahren.

Bei der vorgenannten Reaktortemperatur wird die Umsetzung durch Einleitung von Chlorwasserstoff(3) in ca. 23 Stunden bis zu einem Rest-Cyclopentengehalt von < 1 % durchgeführt.

Das Abgas, überwiegend Chlorwasserstoff und Stickstoff mit wenig Cyclopenten und Cyclopentylchlorid, wird über die Sammelleitung (8) zur Absorption von HCl dem H₂O-Gegenstromwascher (E) und anschließend zur Absorption von Cyclopentylchlorid dem Cyclopentanol-Gegenstromwascher (F) zugeführt.

Das so gereinigte Abgas (9) wird zur Entsorgung einer Abgasverbrennung zugeführt.

Das mit Cyclopentylchlorid angereicherte Cyclopentanol aus dem Gegenstromwascher (F) wird in den Reaktor (A) eingespeist.

Die Prozeßabwässer werden auf einen pH-Wert von < 7 eingestellt und zur Entfernung von organischen Bestandteilen der Stripperkolonne (G) zugeführt. Die obere organische Phase des dort anfallenden Azeotrops (überwiegend Cycloalkylchloride, Cycloolefine und wäßrige Salzsäure) wird in den Reaktor (A) zurückgeführt und die untere wäßrige Azeotropphase in den oberen Teil der Stripperkolonne gefahren.

Der von organischen Anteilen befreite Sumpf (10) wird einer Abwasseraufbereitungsanlage zugeführt.

Nach Reaktionsende werden die Phasen getrennt. Die untere wäßrige Phase wird für den Folgeansatz verwendet bzw. bei Chargenende der Stripperkolonne (G) zugeführt.

Die obere organische Cycloalkylchlorid-Phase wird einer Vakuumtrocknung unterzogen und zur weiteren Aufarbeitung [alkalische Trocknung (C/D) und gegebenenfalls destillative Reinigung] abgefahren.

Das bei der Vakuumtrocknung anfallende Destillat wird dem Folgeansatz zugegeben.

Produktzusammensetzung einer destillierten Ware:

| | |
|---|---|
| Cyclopentan | < 0,05 % |
| Cyclopenten | < 0,05 % |
| Di-cyclopentylether | ≤ 0,001% |
| Cyclopentylchlorid | > 99,5 % |
| APHA-Farbe | < 10 % |
| Ausbeute | 93 % der Theorie |

### Beispiel 2

### Herstellung von Cyclohexylchlorid (Abb. 1)

200 g konzentrierte Salzsäure (ca. 36%ig) und 600 g Cyclohexen (> 99,5%ig) werden unter N₂-Abdeckung in den Reaktor (A) eingefüllt und die Reaktion bei einer Reaktionstemperatur von 40 °C in ca. 20 Stunden analog zu Beispiel 1 durchgeführt.

Der Gegenstromwascher (F) wird mit Cyclohexanol betrieben. Nach erfolgter Phasentrennung und Vakuumtrocknung wird nach GC-Analyse ein > 99%iges Cyclohexylchlorid mit einer APHA-Farbzahl von ca. 20 bis 30 erhalten.

Durch eine einfach Flash-Destillation am Kurzweg kann die APHA-Farbzahl auf ≤ 10 verbessert werden. Die Ausbeute beträgt > 95 % der Theorie.

### Beispiel 3

### Herstellung von Cyclooctylchlorid (Abb. 1)

100 g konzentrierte Salzsäure (ca. 36%ig) und 411 g Cycloocten (96,4%ig, 3,6 % Cyclooctan) werden unter N₂-Abdeckung in den Reaktor (A) eingefüllt und die Reaktion bei einer Reaktortemperatur von 40 °C in ca. 95 Stunden analog zu Beispiel 1 durchgeführt.

Der Gegenstromwascher (F) wird mit Cyclooctanol betrieben. Nach erfolgter Phasentrennung und Vakuumtrocknung wird nach 13 C-NMR-Spektrum 98,9%iges Cyclooctylchlorid mit einer APHA-Farbzahl von ca. 50 erhalten.

Durch eine einfache Flash-Destillation am Kurzweg wird die APHA-Farbzahl auf ≤ 10 verbessert. Die Ausbeute beträgt > 95 % der Theorie.

## Patentansprüche

1. Verfahren zur Herstellung von Alkylhalogeniden aus cycloaliphatischen Olefinen mit 5 bis 16 C-Atomen und konzentrierter Halogenwasserstoffsäure,
dadurch gekennzeichnet,
daß man die Reaktion ohne Katalysatorzusatz und ohne Lösemittel bei einer Temperatur unterhalb des Siedepunkts des Olefins durchführt.

2. Verfahren nach Anspruch 1,
dadurch gekennzeichnet,
daß die Olefine 5 bis 10 C-Atome aufweisen.

3. Verfahren nach Anspruch 1
dadurch gekennzeichnet,
daß die Reaktionstemperatur bei 0 bis 110 °C liegt.

4. Verfahren nach Anspruch 1,
dadurch gekennzeichnet,
daß Alkylchloride hergestellt werden.

## Claims

1. A process for preparing alkyl halides from cycloaliphatic olefins having 5 to 16 C atoms and concentrated hydrohalic acid, characterized in that the reaction is carried out at a temperature below the boiling point of the olefin without addition of catalyst and without solvent.

2. A process according to claim 1, characterized in that the olefins have 5 to 10 C atoms.

3. A process according to claim 1, characterized in that the reaction temperature is 0 to 110°C.

4. A process according to claim 1, characterized in that alkyl chlorides are prepared.

## Revendications

1. Procédé de préparation d'halogénures d'alkyle à partir d'oléfines cycloaliphatiques ayant de 5 à 16 atomes de carbone et d'acide halogénohydrique concentré,
caractérisé en ce qu'
on effectue la réaction sans ajout de catalyseur et sans agent dissolvant, à une température en dessous du point d'ébullition de l'oléfine.

2. Procédé selon la revendication 1,
caractérisé en ce que
les oléfines possèdent de 5 à 10 atomes de carbone.

3. Procédé selon la revendication 1,
caractérisé en ce que
la température de réaction se situe de 0 à 110°C.

4. Procédé selon la revendication 1,
caractérisé en ce que
des chlorures d'alkyle sont préparés.
